# EUROPEAN PATENT SPECIFICATION

(11) **EP 2 792 256 B1**
(45) Date of publication and mention of the grant of the patent: **14.06.2017**
(21) Application number: 12866687.2
(22) Date of filing: 27.01.2012
(51) Int. Cl.: A24F 47/00, A61M 15/06

(54) **FLAVOUR CARTRIDGE AND NON-HEATING TYPE FLAVOUR INHALER**
GESCHMACKSKARTUSCHE UND NICHTWÄRMENDER GESCHMACKSINHALATOR
CARTOUCHE AROMATIQUE ET INHALATEUR D'ARÔME DE TYPE NON CHAUFFANT

(43) Date of publication of application: 22.10.2014
(73) Proprietor: Japan Tobacco, Inc., Tokyo 105-8422 (JP)
(72) Inventor: WATANABE, Tomoichi, Tokyo 130-8603 (JP); YAMADA, Manabu, Tokyo 130-8603 (JP)
(74) Representative: Isarpatent
(86) International application number: PCT/JP2012/051802
(87) International publication number: WO 2013/111320

(56) References cited:
- EP-A1- 2 399 637
- EP-A1- 2 399 638
- EP-A1- 2 609 822
- WO-A1-2010/095660
- JP-A- H02 171 174
- US-A- 3 320 953

## Description

### Technical Field

The present invention relates to a flavour cartridge and a non-heating type flavour inhaler which provide a flavour component to a user through inhalation without requiring ignition.

### Background Art

This type of non-heating type flavour inhaler is disclosed in the following Patent Document 1, for example. This flavour inhaler in Patent Document 1 is provided with a holder and a flavour cartridge held in the holder. The holder includes an air inlet, a mouthpiece, and an air communication path connecting the air inlet and the mouthpiece, while the flavour cartridge is arranged so as to divide the air communication path to an upstream side portion and a downstream side portion in the holder.

In more detail, the flavour cartridge includes a rectangular frame and a flavour pouch held in the frame, and the flavour pouch has a granular material emitting a flavour component and a permeable sheet wrapping the granular material, for example.

According to the above flavour inhaler, when the user inhales through the mouthpiece, an air flow passing through the flavour pouch and then, going toward the mouthpiece is generated in the air communication path. The flavour component emitted from the granular material in the flavour pouch is carried on the above described air flow into the oral cavity of the user and provides the flavour component to the user.

### Prior Art Document

### Patent Document

Patent Document 1: International Patent Publication No. 2010/095660 (WO 2010/095660).
Patent Document 2: European Patent Publication No. EP 2 609 822 A1 relates to a non-heating type flavor inhalator. Patent Document 2 falls under Article 54(3) EPC.

### Summary of the Invention

### Problems to be solved by the Invention

In the case of the flavour inhaler in Patent Document 1, it is not easy to eliminate a gap between an inner surface of the holder and the flavour cartridge or between an inner surface of the frame and the flavour pouch. Thus, such gaps generate a bypass air flow bypassing the flavour pouch as a part of the air flow. Since this bypass flow does not contain the above flavour component, the user cannot effectively taste the flavour component emitted from the granular material.

In order to solve the above described gaps, high molding accuracy of the holder and the flavour cartridge and moreover, a seal structure for preventing the bypass flow and the like are required, which complicates the structure of the flavour inhaler and incurs an increase in its manufacturing cost.

An object of the present invention is to provide a flavour cartridge which can efficiently deliver the flavour component emitted from the granular material and a non-heating type flavour inhaler provided with the flavour cartridge with a simple structure.

### Means for Solving the Problems

The above object is achieved by a flavour cartridge as defined in the independent claim 1. The flavour cartridge comprises: a casing including a longitudinal axis, first and second walls facing each other, and a connection port formed on an outer surface thereof, the first wall having at least one ventilation port;
a flavour pouch accommodated in a compressed state in the casing and extending along the longitudinal axis, the flavour pouch including a granular material emitting a flavour component and a permeable sheet wrapping the granular material; and
a support for supporting the flavour pouch in the compressed state in the casing, the support including a sealing step formed over the whole region of an inner periphery of the casing on an inner surface of the casing and sealingly supporting only the whole periphery of the flavour pouch in close contact with the whole region of an outer periphery of the flavour pouch, and the sealing step ensuring an internal chamber defined between the flavour pouch and the second wall and connected to the connection port.

According to the above flavour cartridge, an air flowing into the casing from the ventilation port reaches the internal chamber through the flavour pouch and generates an air flow flowing out from the internal chamber through the connection port. Such air flow does not bypass the flavour pouch but reliably passes through the flavour pouch and can efficiently contain the flavour component emitted from the granular material. Therefore, if a user can inhale the air flowing out of the connection port, the user can taste the rich flavour component.

Specifically, a space between the first wall and the sealing step is smaller than a thickness of the flavour pouch in a non-load state, and the first wall and the sealing step can maintain the flavour pouch in the compressed state in cooperation with each other.

In this case, the support preferably further includes an abutting element protruding from an inner surface of the second wall and abutting on a surface of the flavour pouch exposed to the internal chamber. More preferably, a top end of the abutting element is substantially positioned in a plane including the sealing step or slightly protrudes from the plane toward the first wall side.

Specifically, the abutting element is a rib extending along the longitudinal axis of the casing or includes a plurality of bosses arranged at intervals from each other along the longitudinal axis.

The above abutting element prevents movement of the granular material in the flavour pouch and maintains density of the granular material uniformly over the whole region of the flavour pouch.

On the other hand, if the flavour pouch is a pillow-packaged product having a traverse seal band at both ends thereof, respectively, the traverse seal bands are preferably folded. Such folding of the traverse seal bands reduces a size of the flavour pouch, and size reduction of the flavour pouch is made possible.

Moreover, the granular material can contain tobacco granules emitting a flavour component specific to tobacco.

Moreover, the present invention provides a non-heating type flavour inhaler, and the flavour inhaler comprises:
the above mentioned flavour cartridge; and
a holder for detachably holding the flavour cartridge, the holder being provided with a hollow mouthpiece having an inner end allowed to connect to the internal chamber of the flavour cartridge through the connection port, in which
the first wall of the flavour cartridge held by the holder and the holder ensure an outside air inlet chamber communicating with an outside air in cooperation with each other.

According to the above described flavour inhaler, when the user inhales through the mouthpiece, an air flow from the outside air inlet chamber to the mouthpiece via the ventilation port, the flavour pouch in the casing, the internal chamber and the connection port is generated.

Specifically, the casing further has a joint protruding from an outer surface thereof, and the joint has the connection port at a tip end thereof and can be inserted into the inner end of the mouthpiece in an airtight manner. In this case, the flavour cartridge is held by the holder and at the same time, connection between the flavour cartridge and the mouthpiece is established.

Moreover, the flavour cartridge can further include a prevention mechanism for determining an attitude of the flavour cartridge allowing insertion of the joint into the inner end of the mouthpiece and preventing misattachment of the flavour cartridge to the holder.

Specifically, if the casing further has an axis extending in the insertion direction of the joint, the prevention mechanism can include the joint having an axis shifted from the axis of the casing or the joint having a point asymmetric outer peripheral shape.

Moreover, the holder has a pipe shape accommodating the flavour cartridge and further has one end having the mouthpiece, the other end, and a slot which extends from the other end toward the one end and makes partial exposure of the outer surface of the flavour cartridge possible, while the flavour cartridge further includes a protrusion exposed from the slot when accommodated in the holder. Such protrusion facilitates removal of the flavour cartridge from the holder.

### Advantageous Effects of the Invention

According to the flavour cartridge and the non-heating type flavour inhaler of the present invention, with a simple structure that the flavour pouch in the flavour cartridge is just maintained in a compressed state, when the user inhales through the mouthpiece, the user can inhale only the air having passed through the flavour pouch. Thus, the air inhaled by the user efficiently contains the flavour component emitted from the granular material, and the user can taste the rich flavour component.

### Brief Description of the Drawings

FIG. 1 is a perspective view of a non-heating type flavour inhaler of one embodiment.
FIG. 2 is an exploded perspective view of the flavour inhaler in FIG. 1.
FIG. 3 is a longitudinal sectional view of the flavour inhaler in FIG. 1.
FIG. 4 is an exploded perspective view of a flavour cartridge.
FIG. 5 is a perspective view of a flavour pouch.
FIG. 6 is a perspective view of the flavour pouch in which traverse seal bands are folded.
FIG. 7 is a longitudinal sectional view of the flavour cartridge.
FIG. 8 is a diagram illustrating a manufacturing process of the flavour cartridge.
FIG. 9 is a view illustrating arrangement of a joint of the flavour cartridge and its shape.
FIG. 10 is a plan view illustrating a lower shell of a variation of the flavour cartridge.
FIG. 11 is a longitudinal sectional view illustrating a flavour inhaler of a variation.
FIG. 12 is an end face view illustrating a distal end of the flavour inhaler in FIG. 11.
FIG. 13 is a longitudinal sectional view illustrating a flavour inhaler of another variation.

### Mode for Carrying out the Invention

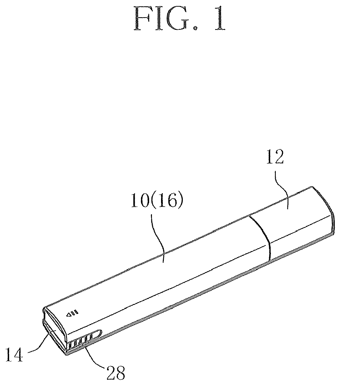

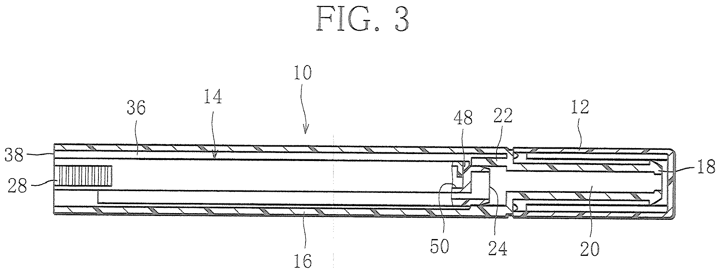

FIGS. 1 to 3 illustrate a non-heating type flavour inhaler of one embodiment, and this inhaler is provided with a holder 10, a cap 12, and a flavour cartridge 14. The holder 10 is integrally molded from a synthetic resin and includes a holder body 16 having a pipe shape and a mouthpiece 18. The holder body 16 has an elliptic-shaped outer peripheral surface when seen on a section thereof. The mouthpiece 18 extends from one end of the holder body 16 and has an internal passage 20 as illustrated in FIG. 3. An inner end of this internal passage 20 is connected into the holder body 16.

In detail, the mouthpiece 18 has a connection port 22 providing an inner end thereof, and the connection port 22 protrudes from one end of the holder body 16 into the holder body 16 and opens toward the other end of the holder body 16. The connection port 22 has an elliptic inner periphery.

The cap 12 is also formed of a synthetic resin and can be detachably attached to the mouthpiece 18.

On the other hand, the flavour cartridge 14 has a thin rectangular stick shape and is detachably accommodated in the holder body 16. That is, the flavour cartridge 14 has a size that can be inserted from the other end of the holder body 16 into the holder body 16. From an inner end of the flavour cartridge 14, a hollow joint 24 is integrally protruded, and the joint 24 provides a connection port at a tip end thereof. When the flavour cartridge 14 is completely inserted into the holder body 16, the joint 24 is inserted into the above described connection port 22, while an outer end of the flavour cartridge 14 is exposed at the other end of the holder body 16.

The joint 24 has an outer peripheral shape that can conform to an inner peripheral shape of the connection port 22 and thus, only by being inserted into the connection port 22, the joint 24 is connected to the connection port 22 in an airtight manner. That is, the flavour cartridge 14 is held in the holder body 16 by inserting the joint 24 into the connection port 22.

Moreover, the holder body 16 has a slot 26 on both side surfaces thereof, respectively. These slots 26 are opened on the other end of the holder body 16 and extend for a predetermined length from the other end toward the one end. On the other hand, the flavour cartridge 14 also has a projection 28 on both side surfaces thereof. Each of projections 28 extends from an outer end toward an inner end of the flavour cartridge 14 to the same degree as a length of the slot 26, has a plurality of grooves on an outer surface thereof, and these grooves increase a friction coefficient of the outer surface of the projection 28.

The above described slot 26 and the projection 28 may be arranged only on one side of the holder body 16 and the flavour cartridge 14.

When the flavour cartridge 14 is accommodated in the holder body 16, the projection 28 is positioned in the slot 26 on the corresponding side and is exposed through the slot 26. Thus, when the flavour cartridge 14 is to be removed from the holder body 16, the user can easily pull out the flavour cartridge 14 from the holder body 16 by pinching the flavour cartridge 14 by fingers at the left and right projections 28.

Moreover, the other end of the holder body 16 has a mark 30 drawn on an upper surface thereof, and the mark 30 indicates that the other end of the holder body 16 is a receiving port allowing insertion of the flavour cartridge 14. On the other hand, the inner end and the outer end of the flavour cartridge 14 also have marks 32 and 34 drawn on upper surfaces thereof, and the mark 32 indicates an insertion direction of the flavour cartridge 14, while the mark 34 indicates a pulling-out direction of the flavour cartridge 14.

As is obvious from FIG. 3, when the flavour cartridge 14 is completely accommodated in the holder body 16, an outside air inlet chamber 36 is ensured between an inner surface of the holder body 16 and an upper surface of the flavour cartridge 14. This outside air inlet chamber 36 extends in a longitudinal direction of the flavour cartridge 14 and is opened as an outside air inlet 38 on the other end of the holder body 16.

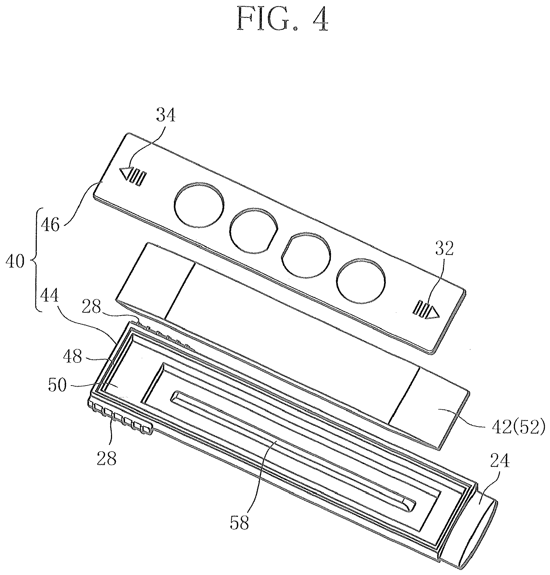

FIG. 4 illustrates a structure of the above described flavour cartridge 14 in detail.

The flavour cartridge 14 includes a casing 40 having a longitudinal axis, and the casing 40 accommodates a flavour pouch 42 as a flavour generation source, and the flavour pouch 42 extends along the longitudinal axis. The casing 40 has a lower shell 44 and a top plate (first wall) 46, and the lower shell 44 and the top plate 46 are both formed of a synthetic resin. The lower shell 44 has an open top, the above described joint 24, and projections 28, and the top plate 46 has the above described marks 32 and 34.

In detail, the lower shell 44 has an upper step 48 on a top thereof, and the upper step 48 extends over the entire periphery of the top. Moreover, the lower shell 44 has a support, that is, a sealing step 50 between its bottom (second wall) and the upper step 48, and the sealing step 50 extends over the entire region of an inner periphery of the lower shell 44. The sealing step 50 divides the inside of the lower shell into an upper region and a lower region.

The above described flavour pouch 42 includes a granular material (not shown) and a permeable sheet 52 wrapping the granular material. As the granular material, tobacco granules can be wrapped, for example. The tobacco granules are particles obtained by shredding or crushing tobacco leaves and stems or powders obtained by treating the particles and emit a flavour component specific to tobacco. Moreover, the permeable sheet is formed of a nonwoven fabric, for example.

The granular material can further include particles of flavors such as menthol other than tobacco granules and furthermore can include flavour emitting particles formed from herbs, tea and the like emitting flavour components other than the flavour component specific to tobacco.

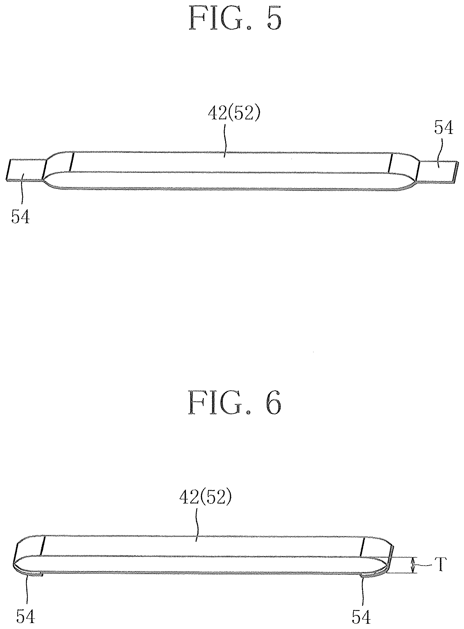

The above described flavour pouch 42 is manufactured by a vertical pillow packaging machine and has traverse seal bands 54 protruded from both ends thereof, respectively, as illustrated in FIG. 5. Before the flavour pouch 42 is accommodated in the upper region of the lower shell 44, the traverse seal bands 54 are folded as illustrated in FIG. 6 and overlapped with the flavour pouch 42.

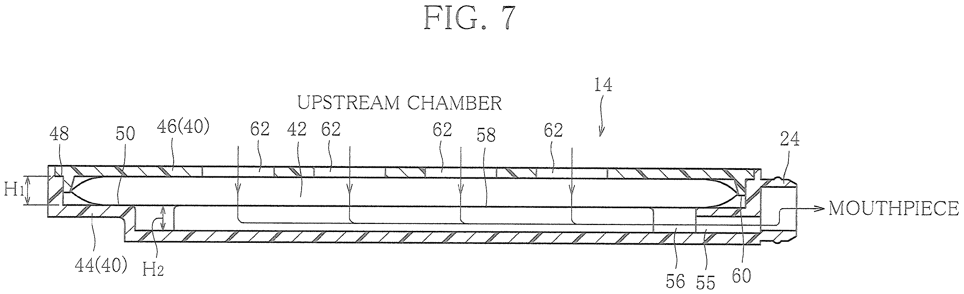

As illustrated in FIG. 7, the flavour pouch 42 is accommodated in the upper region of the lower shell 44, but a substantial height H1 of the upper region, that is, a distance between the sealing step 50 and the upper step 48 is lower than a thickness T (see FIG. 6) of the flavour pouch 42 immediately after the flavour pouch 42 is manufactured.

Moreover, as illustrated in FIG. 7, a communication hole 55 for having the above described joint 24 and the lower region communicate with each other is formed in the lower shell 44. Thus, when the flavour cartridge 14 is accommodated in the holder body 16, and the joint 24 of the flavour cartridge 14 is connected to the connection port 22 of the holder body 16 in an airtight manner, the lower region communicates with the mouthpiece 18. Considering a communication path for an air from the above described outside air inlet chamber 36 through the flavour cartridge 14 to the mouthpiece 18, the lower region provides an internal chamber 56 communicating with the outside air inlet chamber 36 through the upper region.

Moreover, on the bottom of the lower shell 44, a rib 58 as an abutting element is integrally formed at a center thereof. This rib 58 extends in the longitudinal direction of the lower shell 44 while leaving both end regions of the internal chamber 56. Thus, the rib 58 forms the internal chamber 56 annularly. A height H2 of the rib 58 from the bottom of the lower shell 44 is equal to a depth of the internal chamber 56 or larger than the depth. Thus, a top of the rib 58 is positioned on the plane including the sealing step 50 or slightly protrudes from the plane into the upper region.

In the upper region, the flavour pouch 42 is arranged, and at this time, the two traverse seal bands 54 of the flavour pouch 42 have been already folded, and the outer peripheral portion of the flavour pouch 42 is supported by the sealing step 50 over the whole region, and moreover, the center of the flavour pouch 42 is supported by the rib 58.

On the other hand, the top plate 46 is fitted with the open top of the lower shell 44 and is supported by the upper step 48 of the lower shell 44. A thickness of the top plate 46 is equal to a height from the upper step 48 to the top of the lower shell 44. Thus, an upper surface and a lower surface of the top plate 46 are positioned flush with the top of the lower shell 44 and the upper step 48, respectively.

Moreover, a rectangular inner rim 60 is protruded from the lower surface of the top plate 46, and this inner rim 60 is fitted with an inner peripheral surface of the upper region. Thus, the outer peripheral surface of the inner rim 60 is in close contact with the inner peripheral surface of the upper region.

On the top plate 46, four openings 62, for example, are formed. These openings 62 are juxtaposed at intervals from each other in the longitudinal direction of the flavour cartridge 14 and are positioned above the rib 58.

An outer peripheral edge of the top plate 46 is firmly bonded to the upper step 48 of the lower shell 44 over the whole region thereof by an ultrasonic deposition or an adhesive. Thus, a space between the outer peripheral edge of the top plate 46 and the upper step 48 is sealed airtightly.

As described above, the height H1 of the upper region is smaller than the thickness T of the flavour pouch 42. Thus, when the top plate 46 is attached to the open top of the lower shell 44, the top plate 46 presses the flavour pouch 42 on the sealing step 50 and the rib 58 and thus, the flavour pouch 42 is compressed. Such compression of the flavour pouch 42 brings the outer peripheral portion of the flavour pouch 42 into close contact with the sealing step 50 and brings the upper surface of the flavour pouch 42 into close contact with the lower surface of the top plate 46. Thus, a gap between the outer peripheral portion of the flavour pouch 42 and the sealing step 50 is sealed airtightly, and a gap between the lower surface of the top plate 46 and the upper surface of the flavour pouch 42 is also sealed airtightly.

When the user inhales through the mouthpiece 18, as indicated by an arrow in FIG. 7, the air in the outside air inlet chamber 36 of the holder body 16 flows into the flavour pouch 42 through the openings 62 of the top plate 46, passes through the flavour pouch 42 and then, reaches the internal chamber 56. That is, the air in the outside air inlet chamber 36 can reach the internal chamber 56 without bypassing the flavour pouch 42. Therefore, since the air flowing out into the internal chamber 56 passes through the flavour pouch 42 without fail, the flavour component specific to tobacco emitted from the granular material of the flavour pouch 42 is efficiently contained.

After that, the air containing the flavour component is led into the oral cavity of the user from the internal chamber 56 through the communication hole 55, the joint 24, and the mouthpiece 18 and thus, the user can taste the rich flavour component.

As described above, the lower surface of the flavour pouch 42 is pressed onto the above described rib 58 and thus, such rib 58 prevents movement of the granular material in the flavour pouch 42 and maintains the density of the granular material in the flavour pouch 42 uniform. Thus, the air passing through the flavour pouch 42 can be effectively brought into contact with the granular material and can contain the flavour component emitted from the granular material more efficiently.

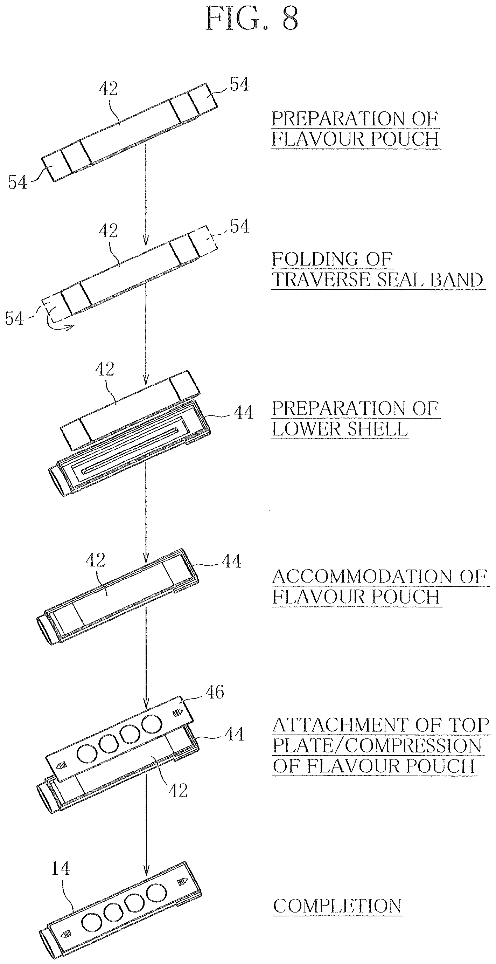

FIG. 8 illustrates a manufacturing process of the flavour cartridge 14, and this manufacturing process includes, as is obvious from the above described explanation, preparation of the flavour pouch 42, folding of the traverse seal bands 54, preparation of the lower shell 44, accommodation of the flavour pouch 42, and mounting of the top plate 46/compression of the flavour pouch 42.

Preferably, the manufacturing process includes a vibration process of vibrating the flavour pouch 42 vertically and/or longitudinally before the flavour pouch 42 is accommodated in the lower shell 44 or before attachment of the top plate 46 after the flavour pouch 42 has been accommodated in the lower shell 44. Such vibration process is helpful in making the density of the granular material in the flavour pouch 42 uniform.

In the vibration process, not only a mechanical vibrator for rotating a cam by a driving source such as an air cylinder or a motor but also an electric vibrator using a piezoelectric element such as an ultrasonic vibrator can be used.

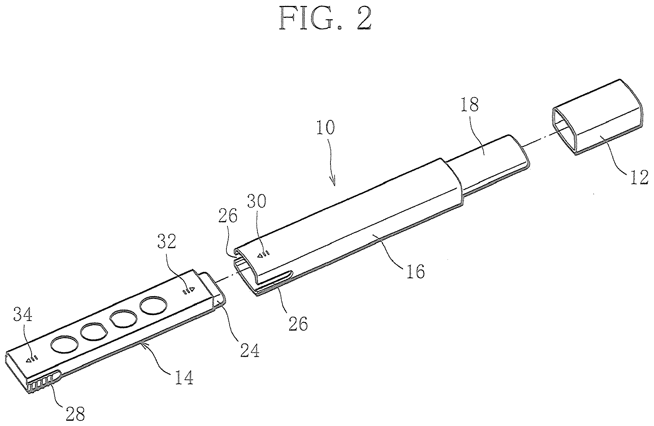

Moreover, according to the flavour inhaler of this embodiment, when the flavour cartridge 14 is inserted into the holder body 16, a vertical direction of the flavour cartridge 14 is important. That is, FIG. 2 illustrates a correct insertion attitude of the flavour cartridge 14, but if the flavour cartridge 14 is inserted into the holder body 16 upside down, the flavour cartridge 14 cannot accurately form the outside air inlet 38 or the outside air inlet chamber 36 in cooperation with the holder body 16, and use itself of the flavour inhaler is made impossible.

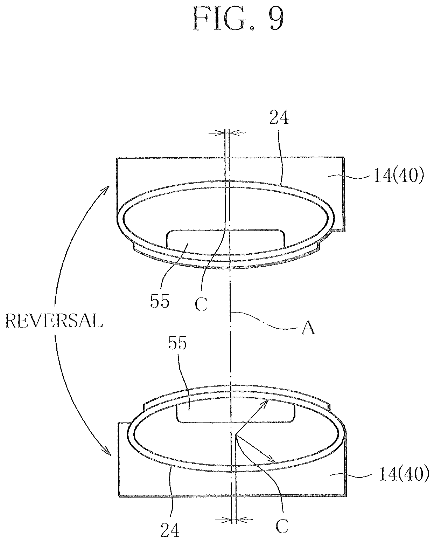

As a mechanism for preventing such misinsertion of the flavour cartridge 14, as illustrated in FIG. 9, a center C of the joint 24 of the flavour cartridge 14 is positioned out of an axis A of the flavour cartridge 14. FIG. 9 illustrates a state in which the center C is shifted in a width direction of the flavour cartridge 14 from the axis A of the flavour cartridge 14, but the center C may be in a state shifted in a thickness direction of the flavour cartridge 14 from the axis A of the flavour cartridge 14.

In an upper side in FIG. 9, the flavour cartridge 14 is illustrated with a correct insertion attitude, while in a lower side in FIG. 9, the flavour cartridge 14 is illustrated in a wrong attitude vertically opposite to the correct insertion attitude. If the flavour cartridge 14 is inserted into the holder body 16 in such wrong attitude,
the joint 24 is shifted in the width direction of the holder body 16 from a normal position where fitting of the holder body 16 into the connection port 22 is allowed, and fitting of the connection port 22 becomes impossible. Thus, the user notices that the insertion attitude of the flavour cartridge 14 is wrong, and after changing the flavour cartridge 14 into the correct insertion attitude, the user can insert the flavour cartridge 14 into the holder body 16.

Instead of shifting the center C of the joint 24 from the axis A of the flavour cartridge 14, the joint 24 may have a point asymmetric outer peripheral shape. Specifically, the joint 24 illustrated in FIG. 9 has an elliptic outer peripheral surface with different oblatenesses at upper and lower portions thereof. Such joint 24 can also prevent misinsertion of the flavour cartridge 14.

The present invention is not limited to the above described embodiment but is capable of various variations.

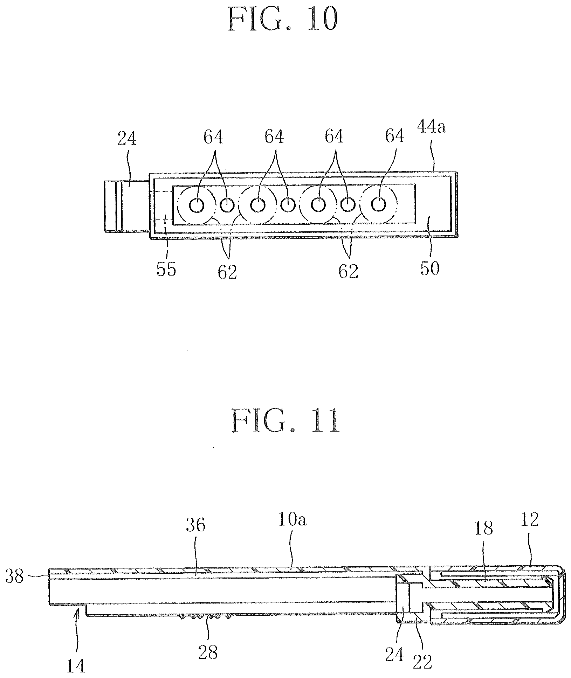

For example, the lower shell 44 can have a plurality of the ribs 58. Moreover, as illustrated in FIG. 10, instead of the rib 58, the lower shell 44 can have a plurality of circular bosses 64. These bosses 64 protrude from the bottom of the lower shell 44 and are juxtaposed in the longitudinal direction of the lower shell 44. Here, some of the bosses 64 are preferably positioned at the centers of the openings 62 in the top plate 46, respectively.

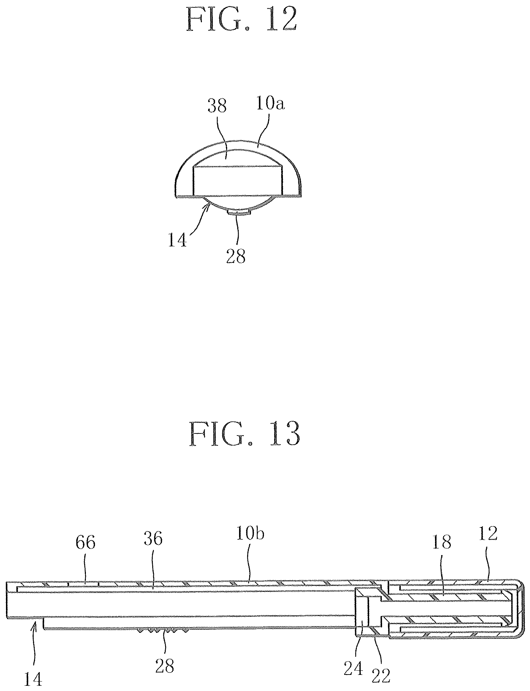

FIGS. 11 and 12 illustrate a flavour inhaler of a variation.

The flavour inhaler of the variation includes a cover-shaped holder 10a instead of the pipe-shaped holder 10. This holder 10a covers an upper half of the flavour cartridge 14 and similarly forms the outside air inlet chamber 36 and the outside air inlet 38 in cooperation with the flavour cartridge 14. In the case of the flavour inhaler of the variation, the above described projection 28 can be arranged on the lower surface of the flavour cartridge 14.

Moreover, FIG. 13 illustrates a flavour inhaler of another variation, and this flavour inhaler includes a cover-shaped holder 10b similarly to the holder 10a. This holder 10b is different from the holder 10a in a point that the holder 10b forms only the outside air inlet chamber 36 in cooperation with the flavour cartridge 14 and has an opening 66 functioning as the outside air inlet 38 on an upper surface thereof.

Moreover, the holder, the cap, the mouthpiece, the casing of the flavour cartridge and the like are not limited to the synthetic resin but it is needless to say that they can be formed of metal, ceramic, lumber and the like.

### Explanation of Reference Signs

- 10: holder
- 14: flavour cartridge
- 16: holder body
- 18: mouthpiece
- 22: connection port
- 24: joint
- 36: outside air inlet chamber
- 38: outside air inlet
- 40: casing
- 42: flavour pouch
- 44: lower shell
- 46: top plate
- 50: sealing step
- 55: communication hole
- 56: internal chamber
- 58: rib
- 62: opening
- 64: boss
- 66: opening

## Claims

1. A flavour cartridge (14) comprising:
a casing (40) including a longitudinal axis (A), first and second walls (44; 46) facing each other, and a connection port formed on an outer surface thereof, the first wall (46) having at least one ventilation port (62) ;
a flavour pouch (42) accommodated in a compressed state in said casing (40) and extending along the longitudinal axis (A), said flavour pouch (42) including a granular material emitting a flavour component and a permeable sheet (52) wrapping the granular material; and
a support for supporting said flavour pouch (42) in the compressed state in said casing (40), said support including a sealing step (50) formed over the whole region of an inner periphery of said casing (40) on an inner surface of said casing (40) and sealingly supporting only the whole periphery of said flavour pouch (42) in close contact with the whole region of an outer periphery of said flavour pouch (42), and the sealing step (50) ensuring an internal chamber (56) defined between said flavour pouch (42) and the second wall (44), and connected to the connection port.

2. The flavour cartridge (14) according to claim 1, wherein
a space between the first wall (46) and the sealing step (50) is smaller than a thickness of said flavour pouch (42) in a non-load state, and the first wall (46) and the sealing step (50) maintain said flavour pouch (42) in the compressed state in cooperation with each other.

3. The flavour cartridge (14) according to claim 2, wherein
said support further includes an abutting element (58; 64) protruding from an inner surface of the second wall (44) and abutting on a surface of said flavour pouch (42) exposed to the internal chamber (56).

4. The flavour cartridge (14) according to claim 3, wherein
a top end of the abutting element (58; 64) is substantially positioned in a plane including the sealing step (50) or slightly protrudes from the plane toward the first wall side.

5. The flavour cartridge (14) according to claim 3, wherein
the abutting element is a rib (58) extending along the longitudinal axis (A).

6. The flavour cartridge (14) according to claim 3, wherein
the abutting element includes a plurality of bosses (64) arranged at intervals from each other along the longitudinal axis (A).

7. The flavour cartridge (14) according to claim 2, wherein
said flavour pouch (42) is a pillow-packaged product having a traverse seal band (54) at both ends thereof, respectively, and the traverse seal bands (54) are folded.

8. The flavour cartridge (14) according to claim 1, wherein
the granular material contains tobacco granules emitting a flavour component specific to tobacco.

9. A non-heating type flavour inhaler comprising:
said flavour cartridge (14) according to claim 1; and
a holder (10) adapted to detachably hold said flavour cartridge (14), said holder (10) including a hollow mouthpiece (18) having an inner end (22) allowed to connect to the internal chamber (56) of the flavour cartridge (14) through the connection port, wherein
the first wall (46) of said flavour cartridge (14) held by said holder (10) and said holder (10) ensure an outside air inlet chamber (36) communicating with an outside air in cooperation with each other.

10. The non-heating type flavour inhaler according to claim 9, wherein
said casing (40) further has a joint (24) protruding from an outer surface thereof, and the joint (24) has the connection port at a tip end thereof and can be inserted into the inner end of the mouthpiece (18) in an airtight manner.

11. The non-heating type flavour inhaler according to claim 10, wherein
said flavour cartridge (14) further includes a prevention mechanism (A, C) adapted to determine an attitude of said flavour cartridge (14) allowing insertion of the joint (24) into the inner end of the mouthpiece (18) and to prevent misattachment of said flavour cartridge (14) to the holder (10).

12. The non-heating type flavour inhaler according to claim 11, wherein
said prevention mechanism includes the joint (24) having a center (C) shifted from the longitudinal axis (A) of said casing (40).

13. The non-heating type flavour inhaler according to claim 11, wherein
the prevention mechanism includes the joint (24) having a point asymmetric outer peripheral shape.

14. The non-heating type flavour inhaler according to claim 10, wherein
said holder (10) has a pipe shape adapted to accommodate said flavour cartridge (14), one end having the mouthpiece (18), the other end, and a slot (26) which extends from the other end toward the one end and makes partial exposure of an outer surface of said flavour cartridge (14) possible; and
said flavour cartridge (14) further includes a protrusion (28) exposed from the slot (26) when accommodated in said holder (10).

## Patentansprüche

1. Aromakartusche (14), umfassend:
ein Gehäuse (40), das eine Längsachse (A), eine erste und zweite Wand (44; 46), die einander zugewandt sind, und eine Anschlussöffnung, die an einer Außenfläche davon gebildet ist, aufweist, wobei die erste Wand (46) mindestens eine Belüftungsöffnung (62) hat;
einen Aromabeutel (42), der in einem komprimierten Zustand in dem Gehäuse (40) aufgenommen ist und sich entlang der Längsachse (A) erstreckt, wobei der Aromabeutel (42) ein körniges Material, das eine Aromakomponente abgibt, und ein durchlässiges Blatt (52), welches das körnige Material umhüllt, enthält; und
einen Träger zum Tragen des Aromabeutels (42) in dem komprimierten Zustand in dem Gehäuse (40), wobei der Träger einen Abdichtungsrücksprung (50) aufweist, der über den gesamten Bereich eines inneren Umfangs des Gehäuses (40) an einer Innenfläche des Gehäuses (40) gebildet ist und abdichtend den gesamten Umfang des Aromabeutels (42) in engem Kontakt mit dem gesamten Bereich eines äußeren Umfangs des Aromabeutels (42) trägt, und der Abdichtungsrücksprung (50) eine Innenkammer (56) bildet, die zwischen dem Aromabeutel (42) und der zweiten Wand (44) definiert und mit der Anschlussöffnung verbunden ist.

2. Aromakartusche (14) nach Anspruch 1, wobei ein Raum zwischen der ersten Wand (46) und dem Abdichtungsrücksprung (50) kleiner ist als eine Dicke des Aromabeutels (42) in einem ungeladenen Zustand, und die erste Wand (46) und den Abdichtungsrücksprung (50) miteinander zusammenwirkend den Aromabeutel (42) in dem komprimierten Zustand halten.

3. Aromakartusche (14) nach Anspruch 2, wobei der Träger ferner ein Anschlagelement (58; 64) aufweist, das von einer Innenfläche der zweiten Wand (44) vorsteht und an eine Fläche des Aromabeutels (42), die zur Innenkammer (56) freiliegt, stößt.

4. Aromakartusche (14) nach Anspruch 3, wobei ein oberes Ende des Anschlagelements (58; 64) im Wesentlichen in einer Ebene positioniert ist, die der Abdichtungsrücksprung (50) aufweist, oder von der Ebene leicht zur ersten Wandseite vorsteht.

5. Aromakartusche (14) nach Anspruch 3, wobei das Anschlagelement eine Rippe (58) ist, die sich entlang der Längsachse (A) erstreckt.

6. Aromakartusche (14) nach Anspruch 3, wobei das Anschlagelement mehrere Ansätze (64) aufweist, die in Intervallen voneinander entlang der Längsachse (A) angeordnet sind.

7. Aromakartusche (14) nach Anspruch 2, wobei der Aromabeutel (42) ein kissenartig verpacktes Produkt ist, das jeweils an beiden Enden davon ein quergerichtetes Abdichtungsband (54) hat, und die quergerichteten Abdichtungsbänder (54) gefaltet sind.

8. Aromakartusche (14) nach Anspruch 1, wobei das körnige Material Tabakgranulate enthält, die eine Aromakomponente, die für Tabak spezifisch ist, abgeben.

9. Nicht wärmender Aromainhalator, umfassend:
die Aromakartusche (14) nach Anspruch 1; und
einen Halter (10), der dazu eingerichtet ist, die Aromakartusche (14) lösbar zu halten, wobei der Halter (10) ein hohles Mundstück (18) aufweist, das ein inneres Ende (22) hat, das mit der Innenkammer (56) der Aromakartusche (14) durch die Anschlussöffnung verbunden werden kann, wobei
die erste Wand (46) der Aromakartusche (14), die von dem Halter (10) gehalten wird, und der Halter (10) miteinander zusammenwirkend eine äußere Lufteinlasskammer (36) bilden, die mit einer Außenluft verbunden ist.

10. Nicht wärmender Aromainhalator nach Anspruch 9, wobei das Gehäuse (40) ferner ein Verbindungsstück (24) hat, das von einer Außenfläche davon vorsteht, und das Verbindungsstück (24) eine Anschlussöffnung an einem Spitzenende davon hat und in das innere Ende des Mundstücks (18) luftdicht eingesetzt werden kann.

11. Nicht wärmender Aromainhalator nach Anspruch 10, wobei die Aromakartusche (14) ferner einen Vertauschsicherungsmechanismus (A, C) aufweist, der dazu eingerichtet ist, eine Ausrichtung der Aromakartusche (14) zu bestimmen, wobei ein Einsetzen des Verbindungsstücks (24) in das innere Ende des Mundstücks (18) gestattet wird und ein nicht korrektes Anbringen der Aromakartusche (14) an dem Halter (10) verhindert wird.

12. Nicht wärmender Aromainhalator nach Anspruch 11, wobei der Präventionsmechanismus das Verbindungsstück (24) mit einer Mitte (C) aufweist, die von der Längsachse (A) des Gehäuses (40) verschoben ist.

13. Nicht wärmender Aromainhalator nach Anspruch 11, wobei der Vertauschsicherungsmechanismus das Verbindungsstück (24) mit einer nicht-punktsymmetrischen äußeren Umfangsform aufweist.

14. Nicht wärmender Aromainhalator nach Anspruch 10, wobei der Halter (10) eine Rohrform, die dazu eingerichtet ist, die Aromakartusche (14) aufzunehmen, ein Ende mit dem Mundstück (18), das andere Ende und einen Schlitz (26) hat, der sich von dem anderen Ende zu dem einen Ende erstreckt und ein teilweises Freilegen einer Außenfläche der Aromakartusche (14) ermöglicht; und
die Aromakartusche (14) ferner einen Vorsprung (28) aufweist, der in dem Schlitz (26) freiliegt, wenn sie in dem Halter (10) aufgenommen ist.

## Revendications

1. Cartouche aromatique (14) comprenant :
une enveloppe (40) présentant un axe longitudinal (A), des première et seconde parois (44 ; 46) en regard l'une de l'autre, et un orifice de raccordement formé sur sa surface extérieure, la première paroi (46) comportant au moins un orifice d'aération (62) ;
un sachet d'arôme (42) logé dans un état comprimé dans ladite enveloppe (40) et s'étendant le long de l'axe longitudinal (A), ledit sachet d'arôme (42) incluant une matière granulaire émettant un composant aromatique et une feuille perméable (52) enveloppant la matière granulaire ; et
un support destiné à supporter ledit sachet d'arôme (42) à l'état comprimé dans ladite enveloppe (40), ledit support comportant un décrochement d'étanchéité (50) formé sur toute la région d'une périphérie intérieure de ladite enveloppe (40) sur une surface intérieure de ladite enveloppe (40) et ne supportant de manière étanche que la totalité de la périphérie dudit sachet d'arôme (42) au contact étroit de toute la région d'une périphérie extérieure dudit sachet d'arôme (42), et le décrochement d'étanchéité (50) ménageant une chambre interne (56) définie entre ledit sachet d'arôme (42) et la seconde paroi (44), et reliée à l'orifice de raccordement.

2. Cartouche aromatique (14) selon la revendication 1, dans laquelle :
un espace entre la première paroi (46) et le décrochement d'étanchéité (50) est plus petit qu'une épaisseur dudit sachet d'arôme (42) à vide, et la première paroi (46) ainsi que le décrochement d'étanchéité (50) maintiennent ledit sachet d'arôme (42) à l'état comprimé, en coopération l'un avec l'autre.

3. Cartouche aromatique (14) selon la revendication 2, dans laquelle :
ledit support comporte en outre un élément de butée (58 ; 64) faisant saillie d'une surface intérieure de la seconde paroi (44) et étant en butée contre une surface dudit sachet d'arôme (42) exposée à la chambre interne (56).

4. Cartouche aromatique (14) selon la revendication 3, dans laquelle :
une extrémité supérieure de l'élément de butée (58 ; 64) est positionnée sensiblement dans un plan incluant le décrochement d'étanchéité (50) ou fait légèrement saillie du plan vers le côté première paroi.

5. Cartouche aromatique (14) selon la revendication 3, dans laquelle :
l'élément de butée est une nervure (58) s'étendant le long de l'axe longitudinal (A).

6. Cartouche aromatique (14) selon la revendication 3, dans laquelle :
l'élément de butée comporte une pluralité de bossages (64) disposés à des intervalles les uns des autres, le long de l'axe longitudinal (A).

7. Cartouche aromatique (14) selon la revendication 2, dans laquelle :
ledit sachet aromatique (42) consiste en un produit sous sachet coussin comportant une bande transversale de scellement (54) à ses deux extrémités, respectivement, et les bandes transversales de scellement (54) sont pliées.

8. Cartouche aromatique (14) selon la revendication 1, dans laquelle :
la matière granulaire contient des granulés de tabac émettant un composant aromatique spécifique du tabac.

9. Inhalateur d'arôme de type non chauffant comprenant :
ladite cartouche aromatique (14) selon la revendication 1 ; et
un organe de maintien (10) conçu pour maintenir de façon amovible ladite cartouche aromatique (14), ledit organe de maintien (10) comportant un embout creux (18) présentant une extrémité intérieure (22) pouvant être reliée à la chambre interne (56) de la cartouche aromatique (14) par l'intermédiaire de l'orifice de raccordement, dans lequel :
la première paroi (46) de ladite cartouche aromatique (14) maintenue par ledit organe de maintien (10) et ledit organe de maintien (10) ménagent une chambre d'entrée d'air extérieur (36) communiquant avec l'air extérieur, en coopération l'un avec l'autre.

10. Inhalateur d'arôme de type non chauffant selon la revendication 9, dans lequel :
ladite enveloppe comporte en outre un joint (24) faisant saillie de sa surface extérieure, et le joint (24) présente l'orifice de raccordement à un bout d'extrémité de celui-ci et peut être inséré dans l'extrémité intérieure de l'embout (18) de manière étanche à l'air.

11. Inhalateur d'arôme de type non chauffant selon la revendication 10, dans lequel :
ladite cartouche aromatique (14) comporte en outre un mécanisme détrompeur (A, C) conçu pour déterminer une orientation de ladite cartouche aromatique (14) permettant l'insertion du joint (24) dans l'extrémité intérieure de l'embout (18) et pour empêcher une fixation incorrecte de ladite cartouche aromatique (14) à l'organe de maintien (10)

12. Inhalateur d'arôme de type non chauffant selon la revendication 11, dans lequel :
ledit mécanisme détrompeur comporte le joint (24) présentant un centre (C) décalé par rapport à l'axe longitudinal (A) de ladite enveloppe (40).

13. Inhalateur d'arôme de type non chauffant selon la revendication 11, dans lequel :
le mécanisme détrompeur comporte le joint (24) ayant une forme périphérique extérieure à asymétrie ponctuelle.

14. Inhalateur d'arôme de type non chauffant selon la revendication 10, dans lequel :
ledit organe de maintien (10) présente une forme de tuyau conçue pour loger ladite cartouche aromatique (14), une extrémité comportant l'embout (18), l'autre extrémité, et une fente (26) qui s'étend de l'autre extrémité vers la première extrémité et permet de faire apparaître partiellement une surface extérieure de ladite cartouche aromatique (14) ; et
ladite cartouche aromatique (14) comporte en outre une saillie (28) rendue visible à travers la fente (26), lorsqu'elle est logée dans ledit organe de maintien (10).
